# EUROPEAN PATENT SPECIFICATION

(11) **EP 0 484 769 B1**
(45) Date of publication and mention of the grant of the patent: **23.08.1995**
(21) Application number: 91118268.1
(22) Date of filing: 26.10.1991
(51) Int. Cl.: C07K 5/06, B01D 9/00

(54) **Improved process for crystallizing L-alpha-aspartyl-L-phenylalanine methyl ester from the solution**
Verbessertes Verfahren zur Kristallisierung von Alpha-Aspartyl-L-Phenylalaninmethylester aus deren Lösung
Procédé amélioré pour la crystallisation de l'alpha-aspartyl-L-phenylalanine méthyl ester à partir de sa solution

(30) Priority: 05.11.1990 JP 297119/90
(43) Date of publication of application: 13.05.1992
(73) Proprietor: MITSUI TOATSU CHEMICALS, Inc., Chiyoda-Ku Tokyo 100 (JP)
(72) Inventor: Ichiki, Hiroshi, Ohmuta-shi, Fukuoka-ken (JP); Taneda, Ryoichi, Ohmuta-shi, Fukuoka-ken (JP); Itoh, Hiroyuki, Ohmuta-shi, Fukuoka-ken (JP); Kono, Yoshitsugu, Ohmuta-shi, Fukuoka-ken (JP)
(74) Representative: Schüler, Horst, Dr.

(56) References cited:
- GB-A- 1 489 028
- US-A- 2 337 317
- CHEMICAL ABSTRACTS, vol. 111, no. 11, 11 September 1989, Columbus, Ohio, US; abstract no. 95773B, S KISHIMOTO ET AL.: 'crystallizers development for the bundle aspartame crystals' page 614 ;column LEFT ;
- Webster's 3rd new international Dictionary of English Language, Merriam-Webster public. Springfield (MA, USA) page 162

## Description

### Background of the Invention:

### 1. Field of the Invention:

This invention relates to a crystallization process of L-α-aspartyl-L-phenylalanine methyl ester which is easy to handle in the filtration and dehydration operations.

### 2. Description of the Related Art:

L-α-aspartyl-L-phenylalanine methyl ester (hereinafter referred simply to as to APM) is a substance expected to use widely as a low-caloric sweetening agent owing to its excellent sweetness. A variety of processes have so far been known to produce APM industrially. Upon execution of any of these processes, however, a crystallization step is indispensable in order to isolate APM from the reaction solution to prepare it as a product.

Generally in the crystallization step, a crude product is dissolved again in water, an organic solvent or a hydrated organic solvent, and the resultant solution is allowed to cool through heat-exchange with a coolant or partial vaporization of the solvent under reduced pressure to deposit the crystals in a crystallization apparatus equipped with stirring blades, the crystals being then filtered or dehydrated by means of a centrifuge or the like. There is also known a process for preparing the crystals which comprises cooling an aqueous APM solution of a certain concentration or higher through conduction heat transfer without giving forced movement such as mechanical agitation to obtain an apparent ice-cream-like pseudo-solid and, if necessary, cooling further the pseudo-solid thus formed.

However, in the case of using crystallization apparatuses equipped with conventional stirrers, APM obtained, for instance, in a crystallization apparatus with a paddle- or turbine-type stirring means presents a fine needle-like crystal habit so that its solid-liquid separation in filtration or dehydration is very insufficient, thus raising serious problems upon practice.

Further, when the solid-liquid separation is repeated, the basal layer of the cake becomes hard by compression and its removal takes a great deal of man-hour. Moreover, in a drying step subsequent to the crystallization step, the larger moisture content of the cake leads to a higher drying load, and the larger bulk specific volume of the resulting dry powder also causes its handling to be very difficult.

A process, in which cooling is effected only by conduction heat transfer without agitation of the system, takes a very long time to operate and hence can hardly be put into industrial practice.

### Summary of the Invention:

The present invention provides a process wherein crystallization of APM from its solution is effected using a crystallization apparatus of a specific structure.

The specific structure of the crystallization apparatus is defined in the Claim . Roughly speaking, the crystallization apparatus is composed of a vessel, a stirrer comprising a revolving shaft and stirring blades, each blade comprising a band plate member capable of sweeping the bottom of the vessel and rod- and band plate-members provided thereon which extend horizontally and vertically, and baffle plates.

### Description of the Drawings:

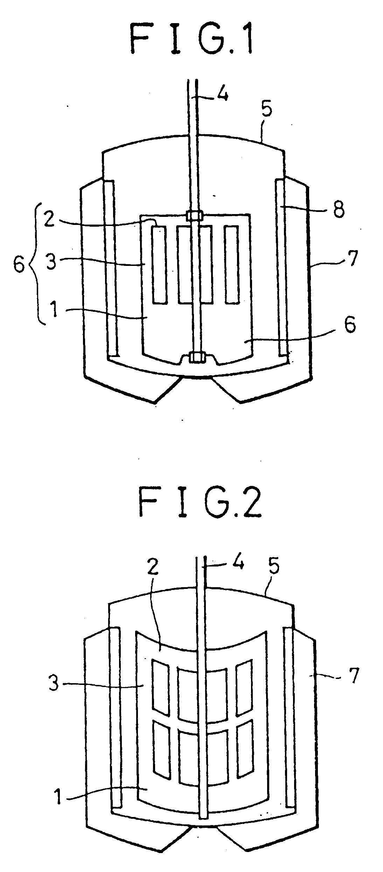

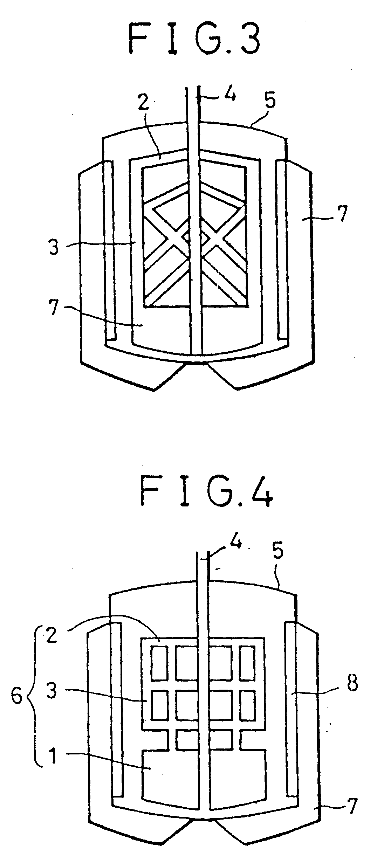

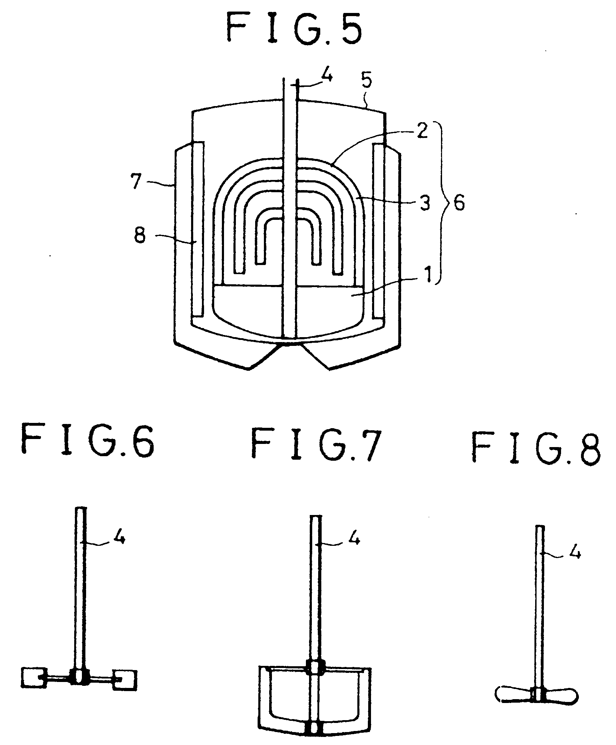

Figs. 1 through 5 show crystallization apparatus having vessel and stirrer with stirring blades useful in the practice of the present invention, and Figs. 6 through 8 illustrate various stirring blades which are not used in the present invention.

Numerals 1, 2, 3 and 4 stand for a first member, a second member, a third member and a revolving shaft, further 5 for vessel, 6 for stirring blade, 7 for jacket and 8 for baffle, respectively.

### Detailed Description of the Invention:

In the present invention, APM is crystallized out under agitation of an APM solution by means of a specific crystallization apparatus as defined in the Claim .

The inner walls of the crystallizer are provided with a plural number of baffle plates vertically from the bottom to the top along the axial direction. APM solutions that can be treated may have a wide range of concentrations. For example, the concentration of APM in aqueous state may range from 2% by weight to the aqueous saturated solution. The concentrations of from 3.5 to 4% by weight may preferably be mentioned.

The crystallization temperature may range from 80°C to 0°C, preferably from 60°C to 5°C. In the crystallization of APM, the solution is allowed to cool slowly while maintaining the peripheral speed of the aforementioned stirring blade at 0.1 - 1.2 m/sec, preferably at 0.3 - 0.6 m/sec.

Cooling of the crystallizing solution is effected by circulating a coolant through a jacket arranged on the outside of the vessel, by means of vacuum evaporation, or by a combination of these means.

Then, the stirring blade used in the present invention will be illustrated. Stirring blades are fixed to a revolving shaft installed in the center of the vessel at one end of each blade. As a matter of course, the stirrer may comprise an odd number, except one, of stirring blades, provided that a smooth rotation of the shaft is assured. However, it is recommended that the stirrer have two stirring blades arranged symmetrically in view of the easiness of fabrication.

A first member constituting the blade is substantially a band plate, sweeping the bottom of the vessel. The space between the lower end of the plate and the bottom of the vessel is preferably as small as possible. Although the reason is not clear, it appears preferable that the liquid flow toward the bottom of the vessel is not large.

It is desirable that there is a certain degree of space between the end of each stirring blade facing the inner walls of the vessel and the inner walls. This is for the purpose of forming liquid flows toward the inner walls.

A second member is practically a rod member provided above the first member and extends from the revolving shaft to the inner walls of the vessel. In other words, it is an arm extending from the revolving shaft. The number of this member is not necessarily limited to one, and therefore a plural number of the members may be disposed in horizontally parallel in a vertical plane. The limitation of the length is the same as that of the first member and can be made shorter. The direction in which the rod member extends may not particularly be parallel to the liquid surface but may be aslant so far as the member is so disposed as to cut across the liquid substantially horizontally.

A third member is a rod- or band plate-member extending substantially vertically in the liquid and agitates the liquid. It may be fixed to only either one or to both of the first and second members, so far as its mechanical strength for agitating the liquid is maintained. No strict limitation is imposed on the direction of the member, and it is sufficient if the member is directed practically vertically. Further, it is enough if the member is installed by way of the first and/or second member so that it can move to cut across the liquid flowing from the inner walls of the vessel to the revolving shaft. A plural number of the members is permitted in a unit stirring blade.

The most common arrangement of the foregoing three members is such that they are disposed in the same vertical plane to form, as a whole, a lattice plate with spaces (voids)in it. However, it is also quite allowable that the plate as a whole constitutes a twistedly curved surface.

The configurations of the stirring blades of the present invention are as illustrated in Figs. 1 to 5. Those shown in figs. 6 to 8 are not to be used in the present invention.

According to the process of the present invention, it is possible to obtain the crystals of good filterability under the agitation of a crystallizing solution. In other words, crystals of APM are deposited continuously while maintaining the liquid flowability satisfactorily, and the crystals thus obtained exhibit good handling properties in the subsequent steps of transportation, separation, drying, etc. and also are very strong to physical impacts.

Generally, the crystals deposited sticks to the heat transfer surface to form so-called "scaling", often causing great difficulty in the removal thereof. However, in the crystallization of APM according to the process of the present invention, it is very easy to remove or take off the crystalline layer from the cooling surface.

Since APM crystals of good filterability can be obtained according to the present invention, remarkable improvements can be achieved not only in the solid-liquid separability of the product, but also in the miniaturization of the apparatus on account of the reduction of crystallization time, in the reduction in the load of the dryer and in the operability of each step. Further, even when the process of the present invention is applied to an APM solution containing impurities such as diketopiperazine (DKP), that is a cyclization product of APM, and L-α-aspartyl-L-phenylalanine, it is possible to obtain APM crystals not containing these impurities because of the reduction in the amount of remaining mother liquor and the improvement in cake washability in the solid-liquid separation step. Thus, the present invention provides an APM crystallization process that is markedly advantageous also from economical viewpoint.

With stirrers of conventional stirring blades as shown in Figs. 6 to 8, as the agitation speed is increased, crystals of APM becomes smaller. On the contrary, when the speed is decreased, the flowability is worsened, causing the crystals to stick to the crystallizer to generate troubles.

The present invention will be described more specifically with reference to the following examples.

### Example 1:

This example was performed by installing a stirrer with stirring blades of Fig. 1 in a crystallizer. A crystallizer 1 is a vertically-cylindrical jacketed vessel with a capacity of 300 liters, which has an inner diameter of 700 mm and a height of 850 mm and is provided with four baffle plates in the interior thereof.

The stirring blades used has a D of 420 mm and an H of 760 mm.

In the crystallizer was charged 250 liters of a starting solution in which 9.5 kg of APM containing 3% of DKP had been dissolved (60°C; the initial concentration of APM was 3.6% by weight). Then, the revolution of the stirring blades was adjusted at 30 rpm and a coolant of 10°C was circulated through a jacket 7. Thus, crystallization was carried out by cooling the solution always from the interior thereof, that is, by controlling the temperature and vacuum inside the crystallizer under reduced pressure by a vacuum pump 4. After about 40 minutes from starting the experiment, crystals began to deposit in the solution. The temperature of the solution at this time was 40°C. After a lapse of one hour, the solution was entirely filled with the crystals. Then, the solution was cooled to 7°C by circulating the coolant of 0°C. The crystallization was completed after three hours from the initiation of the experiment.

The slurry thus obtained was filtered and dehydrated by means of a centrifuge, with the result that the moisture content of the cake was reduced to 40% after 20 minutes. The yield was 12.5 kg (wet basis), the rate of recovery was 80%, and the content of DKP was 0.1%.

### Comparative Example 1:

This comparative example was performed by installing a stirrer with blades of Fig. 6 i.e., blades of flat blade turbine type with D = 400 mm. Crystallization was carried out in the same manner as in Example 1, except that the revolution was set at 100 rpm in view of the characteristics of the stirrin g blades. In the course of crystallization, a portion wherein the slurry did not flow was evolved, and a lot of stickings to the inner walls were found upon discharge. The moisture content was 60% or more even after more than two hours of the subsequent filtration and dehydration. The rate of recovery was 60% because of filtration leakage.

### Example 2:

Using the same stirring blades (D : 420 mm, H : 760 mm) and APM solution as those used in Example 1, crystallization was carried out under atmospheric pressure by regulating the revolution of the stirring blades at 50 rpm and circulating a coolant through the jacket. The temperature of the coolant was 10°C until the crystals began to deposit, and thereafter the coolant of 0°C was circulated. The crystallization was completed when the solution was cooled to 7°C. The whole time of crystallization was about 4 hours.

The slurry thus obtained was filtered and dehydrated by means of a centrifuge, with the result that the moisture content of the cake after 20 minutes was 40%, the yield was 12.5 kg (wet basis), and the rate of recovery was 80%.

Although operations were carried out batchwise in all of the above-described examples, continuous operation is also possible industrially.

### Merits of the Invention:

As is clear from the foregoing description and examples, the present invention has merits in the following respects in the steps of crystallizing and separating APM.
(1) It is possible to employ a conventional vertically-cylindrical vessel, without need for particular equipment except for the stirring blades.
(2) With the slurry containing APM crystals obtained according to the process of the present invention, solid-liquid separation can be effected in a shorter time than with those derived from conventional processes.
(3) Since the separability of crystals is remarkably improved, higher washing effects are achieved and hence products with little impurities can be obtained.
(4) The load in the drying step can be reduced, and good handling properties of the dry powder can be expected.

## Claims

1. A process for crystallizing L-α-aspartyl-L-phenylalanine methyl ester from a solution thereof, which process comprises stirring the solution in a crystallization apparatus provided with a stirrer with stirring blades having structures specified as followings (i) to (v) and baffle plates
(i) at least one member of each blade being fixed to one side of the revolving shaft so that the blade can agitate the solution in the crystallizer,
(ii) each blade comprising at least the following three members,
(iii) a first member which is a band plate and extends laterally to the revolving shaft and is fixed at one end of it, the end which is opposite to said fixed lateral end faces the walls of the vessel extending until it leaves a certain larger space from the walls, the member keeping a certain smaller space from the bottom of the vessel in the downward direction,
(iv) a rod-like second member which is disposed on the side nearer to the liquid surface than the first member and extends horizontally between the revolving shaft and the inner walls of the vessel in the same vertical plane as of the first member, one end thereof being fixed to the revolving shaft,
(v) a plural number of rod- or band plate-like third members, which are installed on the side nearer to the liquid surface than the first member and invariably fixed to at least either one of the first and second members, extending vertically in a perpendicular plane to the liquid surface, and
(vi) a plural number of baffle plates being arranged at intervals vertically from the bottom to the top along the axial direction on the inner walls of the vessel.

## Patentansprüche

1. Ein Verfahren zum Kristallisieren von L-α-Aspartyl-L-phenylalaninmethylester aus einer Lösung von ihm, wobei das Verfahren umfaßt, daß die Lösung in einer Kristallisationsapparatur gerührt wird, die mit einem Rührwerk mit Rührblättern, die die im folgenden in (i) bis (v) spezifizierten Strukturen aufweisen, und mit Prallplatten ausgestattet ist, wobei
(i) wenigstens ein Glied von jedem Rührblatt an einer Seite der Drehwelle so befestigt ist, daß das Blatt die Lösung in der Kristallisationsapparatur rühren kann,
(ii) jedes Blatt wenigstens die folgenden drei Glieder umfaßt,
(iii) ein erstes Glied, das eine bandartige Platte ist und sich seitlich von der Umdrehungswelle erstreckt und an seinem einen Ende befestigt ist, wobei das Ende, das diesem befestigten seitlichen Ende entgegengesetzt ist, den Wänden des Gefäßes gegenüberliegt und sich so weit erstreckt, daß es einen bestimmten größeren freien Raum von den Wänden freiläßt, und das Glied einen gewissen kleineren freien Raum von dem Boden des Gefäßes in der Richtung nach unten freiläßt,
(iv) ein stabartiges zweites Glied, das auf der Seite näher zu der Flüssigkeitsoberfläche als das erste Glied angeordnet ist und sich horizontal zwischen der Umdrehungswelle und den inneren Wänden des Gefäßes in der gleichen vertikalen Ebene wie das erste Glied erstreckt, wobei ein Ende von ihm an der Umdrehungswelle befestigt ist,
(v) eine Vielzahl von stab- oder bandplattenartigen dritten Gliedern, die auf der Seite näher zu der Flüssigkeitsoberfläche als das erste Glied angeordnet sind und unveränderlich wenigstens an einem von dem ersten und dem zweiten Glied befestigt sind und sich vertikal in einer senkrechten Ebene zu der Flüssigkeitsoberfläche erstrecken, und
(vi) eine Vielzahl von Prallplatten in Intervallen vertikal von dem Boden zu dem oberen Teil entlang der axialen Richtung auf den Innenwänden des Gefäßes angeordnet ist.

## Revendications

1. Procédé pour la cristallisation de L-α-aspartyl-L-phénylalanine méthylester à partir de sa solution, lequel procédé comprend l'agitation de la solution dans un appareil de cristallisation équipé d'un agitateur avec des pales d'agitation présentant les structures spécifiées comme étant les suivantes (i) à (v) et des chicanes ou déflecteurs
(i) au moins un élément de chaque pale étant fixé sur un côté de l'arbre tournant de sorte que la pale peut agiter la solution dans le cristalliseur,
(ii) chaque pale comprenant au moins les trois éléments suivants,
(iii) un premier élément qui est une plaquette et s'étend latéralement sur l'arbre tournant et qui est fixé sur une de ses extrémités, l'extrémité qui est opposée à l'extrémité latérale fixe se situe en regard des parois du récipient s'étendant de façon à laisser un espace plus grand à partir des parois, l'élément maintenant un certain espace plus petit à partir du fond du récipient dans la direction vers le bas,
(iv) un second élément en forme de tige qui est disposé sur le côté plus proche de la surface liquide que le premier élément et s'étend horizontalement entre l'arbre tournant et les parois internes du récipient dans le même plan vertical que le premier élément, une de ses extrémités étant fixée sur l'arbre tournant,
(v) une pluralité de troisième éléments en forme de tiges ou de plaquettes montés sur le côté plus proche de la surface liquide que le premier élément et qui sont fixés invariablement sur au moins l'un ou l'autre des premier et second éléments, s'étendant verticalement dans un plan perpendiculaire à la surface liquide, et
(vi) une pluralité de chicanes ou déflecteurs disposés selon des intervalles verticalement à partir du fond jusqu'au sommet le long de la direction axiale sur les parois internes du récipient.
